(19) Europäisches Patentamt
European Patent Office
Office européen des brevets

(11) Publication number: 0 127 217
B1

(12) EUROPEAN PATENT SPECIFICATION

(45) Date of publication of patent specification: 02.11.88

(51) Int. Cl.[4]: C 07 J 13/00, C 07 J 41/00

(21) Application number: 84200597.7

(22) Date of filing: 27.04.84

# (54) New process for the preparation of 20-keto-delta16-steroids and new intermediate compounds formed in this process.

(30) Priority: 29.04.83 EP 83200617

(43) Date of publication of application:
05.12.84 Bulletin 84/49

(45) Publication of the grant of the patent:
02.11.88 Bulletin 88/44

(84) Designated Contracting States:
AT BE CH DE FR GB IT LI LU NL SE

(58) References cited:
EP-A-0 061 416
GB-A-2 079 756
US-A-2 420 489
US-A-2 705 719
US-A-2 884 418
HELVETICA CHIMICA ACTA, vol. 27, 1944, pages 1803-1814, Basel, CH; A. WETTSTEIN: "Über Steroide. Zur Herstellung von 16-Methyl-progesteron und verwandten Verbindungen"
RECUEIL DES TRAVAUX CHIMIQUES DES PAYS BAS, vol. 74, no. 11, November 1955, pages 1465-1466, Amsterdam, NL; S.A. SZPILFOGEL et al.: "Dehydration of 17alpha-hydroxy-20-ketopregnanes"

(73) Proprietor: GIST-BROCADES N.V.
Wateringseweg 1 P.O. Box 1
NL-2600 MA Delft (NL)

(72) Inventor: Van Leusen, Albert Mattheus
De Savornin Lohmanlaan 23
NL-9722 HC Groningen (NL)
Inventor: Van Leusen, Adriaan Marinus
Binnensingel 8
NL-9951 GD Winsum (NL)

(74) Representative: Huygens, Arthur Victor, Dr. et al
c/o Gist-Brocades N.V. Patent & Trademarks
Department Wateringseweg 1 PO Box 1
NL-2600 MA Delft (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 127 217 B1

Courier Press, Leamington Spa, England.

**Description**

The invention relates to a new process for the preparation of 20-keto-steroids from 17-(isocyano-sulfonyl-methylene)-steroids whereby simultaneously an unsaturated bond is introduced between $C_{16}$ and $C_{17}$. Furthermore, the invention relates to new intermediate compounds formed in this process and their preparation.

More particularly, the invention relates to a new process for the introduction into steroids of a 17-side chain containing a 20-keto group under the simultaneous formation of an unsaturated bond between $C_{16}$ and $C_{17}$ by reaction of 17-(isocyano-sulfonyl-methylene)-steroids with an alkylating agent to 20-isocyano-20-sulfonyl-delta$^{16}$-steroids, followed by hydrolysis of these latter compounds to 20-keto-delta$^{16}$-steroids. The invention also relates to the intermediate compounds which are formed in this process, i.e. 20-isocyano-20-sulfonyl-delta$^{16}$-steroids and to the ultimately formed 20-keto-delta$^{16}$-steroids, as far as these latter compounds are new.

The preparation of 17-(isocyano-sulfonyl-methylene)-steroids is disclosed in the simultaneously filed patent application No. 84200595 (EP—A—124934), entitled "17-(Isocyano-sulfonyl-methylene)-steroids, 17-(formamido-sulfonyl-methylene)-steroids and their preparation".

Much attention has been given the last years to new, cheap raw materials for the synthesis of pharmacologically active steroids. Therefore, the degradation of the abundant soya bean derived sterols sitosterol and campesterol by microbiological methods into 17-oxo-steroids was extensively investigated. As a result thereof 17-oxo-steroids are readily available now against low prices, which makes these compounds ideal starting materials for the synthesis of pharmacologically active steroids.

Due to the fact that almost all pharmacologically active steroids have a 17-side chain, there is a clear need for chemical synthesis whereby 17-side chains in steroids are introduced, starting from 17-oxo-steroids.

One of the earliest successful attempts to synthesize 20-keto-delta-16-steroids from 17-keto-steroids can be found in Berichte *71*, 1487 (1938) and Berichte *72*, 182 (1939). More recent examples of the introduction of a 17-acetyl group into a 17-keto-steroid are described in J. Org. Chem. *31*, 24 (1966) and Tetrahedron *31*, 2151—2155 (1975).

In the abovementioned simultaneously filed application a process is described for the introduction of a 17-(isocyano-sulfonyl-methylene)-group into a 17-oxo-steroid. The present invention now concerns a process whereby the compounds thus prepared are converted into steroids with a 17-side chain containing a 20-keto group, whereby simultaneously an unsaturated bond is introduced between $C_{16}$ and $C_{17}$. These 20-keto-delta$^{16}$-steroids can be used for the preparation of pharmaceutically active compounds, especially progesteron steroids, by methods known in the art.

The invention comprises a process for the preparation of 20-keto-delta$^{16}$-steroids of formula III:

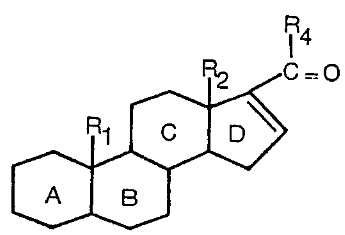

III

in which

$R_1$ represents a hydrogen atom or an alkyl group having 1 to 4 carbon atoms, or is absent in the case of a double bond between $C_{10}$ and $C_1$, $C_5$ or $C_9$,

$R_2$ represents a hydrogen atom or an alkyl group having 1 to 4 carbon atoms,

$R_4$ represents an alkyl group, optionally substituted by one or more halogen atoms, an alkoxy or phenylalkoxy group, a cycloalkyl group having 3—8 carbon atoms or a phenyl group, optionally substituted by one or more halogen atoms, alkyl, alkoxy, nitro or cyano groups and the rings A, B, C and D may contain one or more double bonds and may be substituted by one or more of the following groups: hydroxy, amino, oxo, halogen, alkyl, alkylene, alkoxy, alkoxyalkoxy, epoxy, methylene, alkylenedioxy, alkylenedithio or alkyleneoxythio by reacting a 17-(isocyano-sulfonyl-methylene)-steroid of formula I:

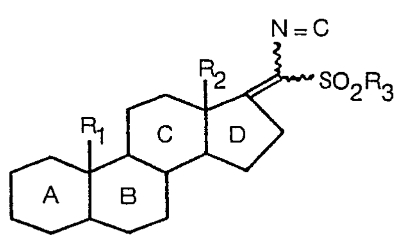

I

in which

$R_1$ and $R_2$ have the meanings as listed above and rings A, B, C and D may contain double bonds and may carry substituents as defined above and

$R_3$ represents an alkyl group, an optionally substituted aryl group or a dialkylamino group, wherein the alkyl groups together with the nitrogen atom may form a heterocyclic ring with up to 8 ring atoms and optionally containing an oxygen atom under basic conditions with an alkylating agent $QR_4$, wherein $R_4$ has the meaning as defined above and Q may be any group which is readily displaced by a nucleophile, followed by hydrolysis in an acidic aqueous solution of the intermediate 20-isocyano-20-sulfonyl-delta$^{16}$-steroids.

The invention comprises also a process for the preparation of 20-isocyano-20-sulfonyl-delta$^{16}$-steroids of formula II

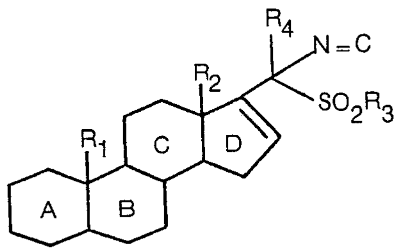

II

in which $R_1$, $R_2$, $R_3$ and $R_4$ have the meanings as defined above, and rings A, B, C and D may contain double bonds and may carry substituents as defined above by reacting a 17-(isocyano-sulfonyl-methylene)-steroid according to claim 1 under basic conditions with an alkylating agent $QR_4$ as defined above.

The invention comprises further the new 20-isocyano-20-sulfonyl-delta$^{16}$-steroids as defined above.

According to another aspect of the invention these compounds are hydrolyzed in an acidic aqueous solution in the 20-keto-delta$^{16}$-steroids as defined above.

More particularly, the invention relates to the above-described processes in which the 17-(isocyano-sulfonyl-methylene)-steroid has the general formula I:

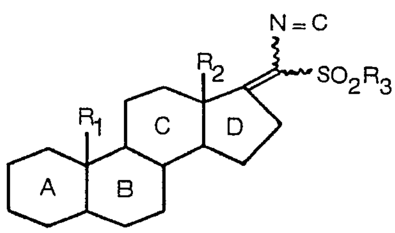

I

and the 20-isocyano-20-sulfonyl-delta$^{16}$-steroid has the general formula

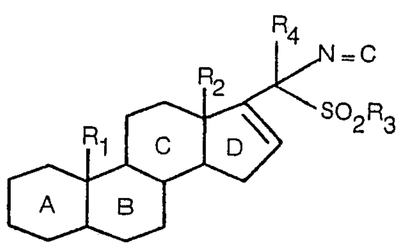

II

in which general formules $R_1$ represents a hydrogen atom or an alkyl group having 1 to 4 carbon atoms, preferably a methyl group, or is absent in the case of a double bond between $C_{10}$ and $C_1$, $C_5$ or $C_9$, $R_2$ represents a hydrogen atom or an alkyl group having 1 to 4 carbon atoms, preferably a methyl group, $R_3$ represents an alkyl or dialkylamino group, or an aryl group substituted or not substituted by one or more halogen atoms or alkyl, alkoxy, nitro or cyano groups, $R_4$ represents an alkyl group optionally containing one or more substituents, and the rings A, B, C and D optionally contain one or more double bonds, are substituted or not substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups or alkylenedioxy, alkylenedithio or alkyleneoxythio groups.

When $R_3$ represents an alkyl group, suitable alkyl groups are straight or branched alkyl groups having 1—10 carbon atoms.

When $R_3$ represents a dialkylamino group, suitable dialkylamino groups are dialkylamino groups wherein the alkyl groups are the same or different and contain 1—8 carbon atoms, preferably 1—4 carbon atoms, or a dialkylamino group wherein the alkyl groups together with the nitrogen atom form a

heterocyclic ring which optionally may contain an oxygen atom, the ring containing up to 8 ring atoms. Preferred dialkyl amino groups are dimethylamino, diethylamino, pyrrolidino and morpholino.

When $R_3$ represents an aryl group, suitable groups are phenyl and naphtyl groups substituted or not substituted by a halogen atom, one or more alkyl groups or an alkoxy group, preferably a phenyl, p-methoxyphenyl or p-methylphenyl group.

When the rings A, B, C and D contain one or more double bonds, these double bonds are preferably present between $C_1$ and $C_2$, $C_3$ and $C_4$, $C_4$ and $C_5$, $C_5$ and $C_6$, $C_6$ and $C_7$, $C_9$ and $C_{10}$, $C_9$ and $C_{11}$ and/or $C_{11}$ and $C_{12}$. More preferably the double bond is present between $C_4$ and $C_5$ and/or $C_9$ and $C_{11}$.

When two or more double bonds are present especially the following systems are preferred: $C_3$—$C_4$ and $C_5$—$C_6$, $C_4$—$C_5$ and $C_6$—$C_7$, $C_1$—$C_2$ and $C_4$—$C_5$, $C_1$—$C_2$, $C_3$—$C_4$ and $C_5$—$C_{10}$ and $C_1$—$C_2$, $C_4$—$C_5$ and $C_6$—$C_7$. Preferably there is also a double bond between $C_9$ and $C_{11}$.

When the rings A, B, C and D are substituted by a hydroxy group, suitable groups are 3-, 9-, 11-, 12-, or 14-hydroxy groups, preferably a 3- or 9-hydroxy group.

When the rings A, B, C and D are substituted by an amino group, suitable amino groups are 3-alkylamino groups, preferably containing 1—4 carbon atoms, 3-dialkylamino groups wherein the alkyl groups are the same or different, each alkyl group preferably containing 1—4 carbon atoms, or amino groups in which the nitrogen atom together with the alkyl groups form a heterocyclic ring, preferably containing 1—8 ring atoms, which ring optionally may contain an oxygen atom. Particularly preferred are dimethylamino, diethylamino, pyrrolidino and morpholino.

When the rings A, B, C and D are substituted by an oxygen atom this oxygen atom is preferably present at $C_3$, $C_{11}$ or $C_{12}$.

When the rings A, B, C and D are substituted by a halogen atom, suitable halogen atoms are 6-, 9- or 11-fluorine, chlorine or bromine atoms, preferably 6- or 9-fluorine or chlorine atoms.

When the rings A, B, C and D are substituted by an alkyl group, suitable alkyl groups are 1-, 2-, 6-, 7- or 16-methyl groups, preferably 1-, 6- or 16-methyl.

When the rings A, B, C and D are substituted by an alkoxy group, suitable alkoxy groups are 3-, 9-, 11- or 12-alkoxy groups containing 1—4 carbon atoms, preferably 3-, 9- or 11-methoxy or ethoxy groups.

When the rings A, B, C and D are substituted by an alkoxyalkoxy group, suitable groups are 3- or 11-methoxymethoxy, methoxyethoxy and tetrahydropyranyloxy groups.

When the rings A, B, C and D are disubstituted, suitable substituents are epoxy groups attached to $C_1$ and $C_2$ or $C_9$ and $C_{11}$ or a methylene group attached to $C_1$ and $C_2$ or a 3,3-alkylenedioxy, a 3,3-alkylenedithio or a 3,3-alkylene oxythio group. The alkylene group preferably contains 2 or 3 carbon atoms.

More particularly the invention relates to the beforementioned processes for the preparation of the compounds as described before in which $R_1$ and $R_2$ represent methyl groups, and which are substituted by a halogenatom, especially fluorine, or a hydroxy group at $C_9$ and a hydroxy or keto group at $C_{11}$, or containing functional groups, as a double bond or epoxy group between $C_9$ and $C_{11}$, which can be converted by methods known in the art into the groups mentioned before, and which contain a keto group at $C_3$ and double bonds between $C_1$ and $C_2$ and/or $C_4$ and $C_5$, or containing functional groups which can be converted into the keto group and double bonds as mentioned before.

All compounds from which it is known that they can be used as alkylating agents may be used in the process of the invention.

Suitable alkylating agents $QR_4$ which can be used in the processes of the invention are those alkylating agents in which $R_4$ represents an alkyl group, preferably having 1—6 carbon atoms, substituted or not substituted by one or more halogen atoms, an alkoxy or phenylalkoxy group, a cycloalkyl group having 3—8 carbon atoms or a phenyl group, substituted or not substituted by one or more halogen atoms or alkyl, alkoxy, nitro or cyano groups. Preferably $R_4$ is methyl, halomethyl, particularly chloro, bromo- and iodomethyl, methoxy or benzyloxy group. The group Q may be any group which is readily displaced by a nucleophile. Suitable groups are bromine, chlorine, iodine, sulphate, quaternary ammonium and sulfonate.

Preferably the alkylating agent is a methylating agent, especially a methyl halogenide as methyl iodide. Other preferred alkylating agents are substituted methyl halogenides wherein the substituent is a halogen, as bromine, chlorine and iodine, or an alkoxy group as methoxy or benzyloxy.

The reaction of the 17-(isocyano-sulfonyl-methylene)-steroid with the alkylating agent is carried out under basic conditions.

Usually the reaction is carried out with a strong alkaline agent in an organic solvent, preferably in an inert gas atmosphere. Examples of useful strong alkaline agents are alkali metal alcoholates such as alkali metal t-butylates and alkali metal ethanolates, alkali metal hydrides, alkali metal amines, alkali metal alkyls and alkali metal aryls, in which the alkali metal is generally lithium, sodium or potassium. Potassium t-butoxide is preferably used.

The reaction is preferably carried out at low temperature, e.g. between −20 and −80°C, preferably between −30 and −60°C, dependant on the solvent used too.

The reaction is further preferably carried out in a polar organic solvent such as tetrahydrofuran, dimethylformamide, 1,2-dimethoxyethane, hexamethylphosphortriamide, dioxane, benzene, toluene or mixtures thereof.Tetrahydrofuran is preferred.

The inert gas atmosphere is preferably a nitrogen or an argon atmosphere.

The reaction can also be carried out in an inert organic solvent to which a base is added. Suitable

organic solvents are methylene chloride, chloroform, 1,2-dichloroethane, benzene, toluene, chlorobenzene, dioxan, bis-(2-methoxyethyl)ether, 1,2-dimethylethane, tetrahydrofuran or mixtures thereof. As the first step and the second step may be performed in a one-pot-reaction preferably a solvent is used in which also the hydrolysis can be carried out. Suitable bases which can be used are metal hydroxides, and quaternary ammonium and phosphonium hydroxides, preferably alkali hydroxides, e.g. potassium hydroxide and quaternary ammonium hydroxides, e.g. triethylbenzylammonium hydroxide. Also alkali alcoholates, e.g. potassium-butoxide may be used. The reaction can be carried out at temperature between 0° and 100°C, prefrably between 0° and 30°C. Sometimes it may be necessary to add a catalyst to the reaction mixture in the form of an quaternary ammonium or phosphonium salt, for instance a trimethylbenzyl ammonium halogenide, a triethylbenzyl ammonium halogenide, a tetrabutyl ammonium halogenide or a alkyl triaryl phosphonium halogenide. Also crown ethers, as 15-crown-5 or 18-crown-6, can be used.

Furthermore the reaction can also be performed using phase transfer-conditions, i.e. a two phase system of an organic layer and an aqueous layer to which a phase transfer catalyst has been added.

See for a general survey of phasetransfer reactions E.V. Dehmlov and S. S. Dehmlov, Phase Transfer Catalysis, Weinheim, Verlag Chemie, 1980.

Suitable organic solvents for the organic layer are methylene chloride, chloroform, 1,2-dichloroethane, benzene, toluene, chlorobenzene and dichlorobenzene. In general all those organic solvents can be used which are immiscible with water, and in which the relevant compounds are soluble.

Suitable aqueous layers are solutions of alkali metal hydroxides in water, for example 5—50% solutions of lithium hydroxide, sodium hydroxide or potassium hydroxide. Suitable phase transfer catalysts are quaternary ammonium and phosphonium salts and crown-ethers, for instance benzyltrialkyl ammonium halogenides, tetraalkyl ammonium halogenides, alkyltriaryl phosphonium halogenides, 15-crown-5 and 18-crown-6.

The hydrolysis of the 20-isocyano-20-sulfonyl-delta$^{16}$-steroids to the 20-keto-delta$^{16}$-steroids can be carried out in an organic solvent, using an acidic aqueous solution. Suitable organic solvents are for instance diethyl ether, methanol and tetrahydrofuran. Suitable acids are diluted strong acids as hydrogen chloride, sulfuric acid and phosphoric acid. Also acetic acid and formic acid can be used.

It is observed that sometimes not only the 20-isocyano-20-sulfonyl group is hydrolyzed, but also other groups linked to the steroid skeleton. These groups may have had the function of protective groups in the preceding reactions.

The invention also relates to the intermediate 20-isocyano-20-sulfonyl-delta$^{16}$-steroids of the general formula:

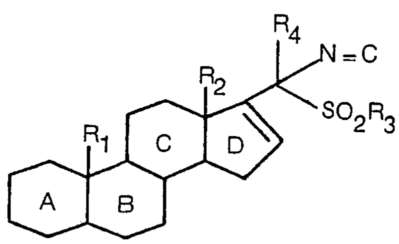

II

wherein the substituents are as defined above.

The invention is illustrated by the following examples. In the examples THF and DME represent tetrahydrofuran, respectively dimethoxyethane. The specific rotation of the compounds was measured using light of the sodium D line.

Example 1a

Preparation of 3-methoxy-20-isocyano-20-p-methylphenylsulfonylpregna-3,5,16-triene

To a solution of 3-methoxy-17-(isocyano-p-methylphenylsulfonyl-methylene)-androsta-3,5-diene (954 mg, 2 mmol) in benzene (40 ml), methyl iodide (0.3 ml, 4.8 mmol), benzyltriethylammonium chloride (40 mg) and 50% aqueous NaOH solution (20 ml) were added. The mixture was stirred vigorously for one hour at 80°C. The organic layer was separated, washed with brine and dried. After evaporation of the solvent the title compound was obtained (910 mg, 93%) m.p. 170—195°C. IR (Nujol*): 2180 (N=C), 1665, 1640, 1625, 1610 (C=C + Ar), 1345, 1170 ($SO_2$) $cm^{-1}$. $^1H$ NMR ($CDCl_3$): delta 0.7—2.8 (m), 1.0 (s), 2.0 (2 x s), 2.49 (s), 3.60 (s, 3H), 5.0—5.4 (m, 2H), 6.20 (m, 1H), 7.32, 7.50, 7.82, 7.98 (ABq, 4H).

Example 1b

Preparation of pregna-4,16-diene-3,20-dione

The compound prepared in Example 1a (246 mg, 0.5 mmol) was dissolved in diethyl ether (50 ml). After addition of 15% aqueous HCl solution (20 ml) the mixture was stirred vigorously at ambient temperature. The organic layer was separated, washed with brine, then with saturated $NaHCO_3$ solution and dried ($Na_2SO_4$). After evaporation the title compound was obtained (130 mg, 70%). Melting point (after

* Trade Mark

chromatography and sublimation) 180°C. $(alpha)^{20}$: +168° (c 1.0, $CHCl_3$) (Litt. A. Wettstein, Helv. Chim. Act. *27*, 1803 (1944); m.p. 186—188°C, $(alpha)^{21}$ +154° (EtOH)).

Example 2

Preparation of 3-methoxy-19-nor-pregna-1,3,5(10),16-tetraen-20-one

3-Methoxy-17-(isocyano-p-methylphenylsulfonyl-methylene)-estra-1,3,5(10)-triene (231 mg, 0.5 mmol) was dissolved in DME (3 ml). The solution was cooled till −40°C under a nitrogen atmosphere. After addition of potassium-t-butoxide (100 mg, 0.8 mmol) methyl iodide (142 mg, 0.6 mmol) was added after 10 minutes. The temperature was slowly raised to +10°C (two hours). The reaction mixture was added to a mixture of diethyl ether (30 ml) and concentrated aqueous hydrogen chloride solution (2 ml), followed by vigorously shaking. After filtration of the organic layer over alumina (act. II—III) and evaporation of the solvent the title compound was obtained (150 mg, 97%, m.p. 180°C). Crystallization from ethyl acetate afforded the title compound with a melting point of 187°C $(alpha)^{22}$: +122°C.

Example 3

Preparation of 3-methoxy-20-isocyano-20-p-methylphenylsulfonyl-21-benzyloxy-19-nor-pregna-1,3,5(10),16-tetraene

3-Methoxy-17-(isocyano-p-methylphenylsulfonyl-methylene)-estra-1,3,5(10)-triene (230 mg, 0.5 mmol) was dissolved in DME (3ml). The solution was cooled under a nitrogen atmosphere till −40°C. Potassium-t-butoxide (100 mg) was added followed by chloromethyl benzyl ether (100 mg, 0.64 mmol) after 10 minutes. The temperature was slowly raised (2.5 hours) to +10°C. The reaction mixture was poured into a saturated solution of $NaHCO_3$, followed by extraction with methylene chloride.

The organic layer was dried and evaporated. The residue was dissolved in methylene chloride and filtrated over alumina (act. II—III). Evaporation of the solvent and crystallization from methanol afforded the title compound (105 mg, 36%), m.p. 153°C (dec.). IR (Nujol*): 2190 (N=C), 1620, 1605, 1585 (Ar + C=C), 1330, 1155 ($SO_2$) $cm^{-1}$. $^1H$ NMR ($CDCl_3$): delta 0.75 (s, 3H), 1.1—3.1 (m), 2.38 (s), 3.7 (s, 3H), 3.99 (s, 0.7H), 4.02 (s, 1.3H), 4.53 (s, 2H), 6.25—6.75 (m, 3H), 6.9—7.4 (m, 8H), 7.64, 7.79 ($\frac{1}{2}$ ABq, 2H). Anal. $C_{36}H_{39}NO_4S$ (581.78), calc. C 74.32; H 6.76; N 2.41; S 5.51; found C 73.8; H 6.8; N 2.3; S 5.4.

The same reaction was also performed in the way as described in Example 1a, however at ambient temperature. Yield after crystallization from methanol: 50%.

The title compound can be hydrolyzed in the way as described in Example 1b to 3-methoxy-21-benzyloxypregna-1,3,5(10),16-tetraen-20-one.

Example 4

Preparation of 3-methoxy-20-isocyano-20-t-butylsulfonylpregna-3,5,16-triene

The title compound was obtained in a low yield according to the process described in Example 1a starting from 3-methoxy-17-(isocyano-t-butylsulfonyl-methylene)androsta-3,5-diene.

Example 5

Preparation of 3-methoxy-20-isocyano-20-methylsulfonylpregna-3,5,16-triene

The title compound was obtained according to the process described in Example 1a starting from 3-methoxy-17-(isocyanomethylsulfonyl-methylene)-androsta-3,5-diene (200 mg). Yield 210 mg. IR (Nujol*): 2180 (N=C), 1660, 1640 (C=C), 1330, 1160 ($SO_2$). $^1H$ NMR ($CDCl_3$): delta 0.7—2.7 (m), 1.0 (s), 1.10 (s), 1.97 (s), 2.00 (s), 3.02 (s, 3H), 3.55 (s, 3H), 5.0—5.4 (m, 2H), 6.1—6.3 (m), 6.4—6.6 (m).

Hydrolysis of the title compound gave pregna-4,16-diene-3,20-dione (35%).

Example 6

Preparation of 3-methoxy-20-isocyano-20-n-decylsulfonylpregna-3,5,16-triene

The title compound was prepared according to the method described in Example 1a starting from 3-methoxy-17-(isocyano-n-decylsulfonyl-methylene)-androsta-3,5-diene (527 mg). IR (Neat) 2160 (N=C), 1655, 1630 (C=C). $^1H$ NMR ($CDCl_3$): delta 0.6—3.5 (m), 1.0 (s), 1.11 (s), 1.3 (s, br), 1.98 (s, br), 3.56 (s, 3H), 5.0—5.3 (m, 2H), 6.05—6.25 (m), 6.3—6.5 (m).

Hydrolysis of the title compound gave pregna-4,16-diene-3,20-dione (45%).

Example 7

Preparation of 3-methoxy-20-isocyano-20-p-methoxyphenylsulfonylpregna-3,5,16-triene

The title compound was prepared according to the method described in Example 1a, starting from 3-methoxy-17-(isocyano-20-p-methoxyphenylsulfonyl-methylene)androsta-3,5-diene, however, using toluene instead of benzene. Yield: 92%. M.p. 160—180°C (dec.). IR (Nujol*): 2160 (N=C), 1660, 1635, 1600, 1585 (C=C), 1335, 1150 ($SO_2$). $^1H$ NMR ($CDCl_3$): delta 0.74—2.77 (m), 0.94 (s), 1.92 (s), 3.55 (s, 3H), 3.88 (s, 3H), 5.04—5.35 (m, 2H), 6.13 (m, 1H), 6.95, 7.09, 7.78, 7.93 (ABq, 4H).

Hydrolysis of the title compounds gave pregna-4,16-diene-3,20-dione (57%).

* Trade Mark

Example 8a

Preparation of 3-methoxy-20-isocyano-20-p-methylphenylsulfonyl-21-benzyloxy-pregna-3,5,16-triene

The title compound was prepared according to the process described in Example 1a starting from 3-methoxy-17-(isocyano-p-methylphenylsulfonyl-methylene)-androsta-3,5-diene (954 mg, 2 mmol) and chloromethyl benzyl ether (470 mg, 3 mmol). The product obtained was purified by filtration over alumina (act. II—III). Crystallization from methanol at −20°C gave 645 mg (54%) of the title compound, m.p. 90—110°C (dec.). IR "Nujol" 2150 (N=C), 1650, 1625, 1590 (C=C), 1325, 1140 ($SO_2$), 1090 (C—O—C). $^1$H NMR ($CDCl_3$): delta 0.67—2.77 (m), 0.76 (s), 0.98 (s), 2.37 (s), 3.53 (s, 3H), 3.77, 3.94, 4.02, 4.19 (q, 2H), 5.54 (s, 2H), 5.03—5.30 (m, 2H), 6.29—6.47 (m, 1H), 6.98—7.47 (m, 7H), 7.76, 7.83 (½AB, 2H).

Example 8b

Preparation of 21-benzyloxy-pregna-4,16-diene-3,20-dione

The compound prepared in Example 8a (448 mg) was hydrolyzed in methylene chloride (15 ml) and diethylether (45 ml) at 0°C for half an hour. Yield: 210 mg containing 75% of the title compound. IR (Nujol*): 1670 (C=O), 1620, 1590 (C=C), 1110 (C—O—C). $^1$H NMR ($CDCl_3$): delta 0.70—2.90 (m), 0.97 (s), 1.22 (s), 4.33 (s, 2H), 4.57 (s, 2H), 5.70 (s, 1H), 6.62—6.82 (m, 1H), 7.19 (s, 5H).

Example 9a

Preparation of 20-isocyano-20-p-methylphenylsulfonyl-pregna-1,4,16-triene-3-one

The title compound was prepared according to the process described in Example 1a starting from 17-(isocyano-p-methylphenylsulfonyl-methylene)-androsta-1,4-diene-3-one (462 mg, 1 mmol). Yield 450 mg (95%), m.p. 160—175°C (dec.)). IR (Nujol*): 2140 (N=C), 1660 (C=O), 1620, 1595 (C=C), 1325, 1150 ($SO_2$). $^1$H NMR ($CDCl_3$): delta 0.70—3.27 (m), 0.92 (s), 1.01 (s), 1.23 (s), 1.91 (s), 2.42 (s), 6.05, 6.08, 6.26, 6.29 (2xd, 2H), 6.92, 7.09 (d, 1H), 7.28, 7.40, 7.73, 7.86 (AB, 4H).

Example 9b

Preparation of pregna-1,4,16-triene-3,20-dione

The compound prepared in Example 9a (450 mg) was hydrolyzed in methylenechloride (15 ml) and diethylether (30 ml) at 20°C for two minutes. The product obtained was purified by filtration over alumina. Yield: 235 mg (75%). Crystallization from methanol gave the title product, m.p. 200—204°C (dec.) (alpha)$^{22}$ +134° ($CHCl_3$, c 1.00). IR (Nujol*): 1680 (C=O), 1630, 1610, 1590 (C=C). $^1$H NMR ($CDCl_3$): delta 0.53—2.86 (m), 0.98 (s), 1.29 (s), 2.27 (S), 6.04, 6.07, 6.24, 6.27 (2xd, 2H), 6.55—6.77 (m, 1H), 6.95—7.11 (d, 1H).

Example 10a

Preparation of 3-methoxy-20-isocyano-20-p-methylphenylsulfonylpregna-3,5,9(11),16-tetraene

The title compound was prepared according to the process described in Example 1a starting from 3-methoxy-17-(isocyano-p-methylphenylsulfonyl-methylene)-androsta-3,5,9(11)-triene (476 mg, 1 mmol). Yield: 460 mg (94%). IR (Nujol*): 2140 (N=C), 1655, 1630, 1595 (C=C), 1320, 1150 $cm^{-1}$ ($SO_2$). $^1$H NMR ($CDCl_3$): delta 0.60—2.88 (m), 0.73 (s), 0.96 (s), 1.17 (s), 1.98 (s), 2.48 (s), 3.58 (s, 3H), 5.07—5.63 (m, 3H), 6.09—6.39 (m, 1H), 7.27, 7.41, 7.76, 7.90 (AB, 4H).

Example 10b

Preparation of pregna-4,9(11),16-triene-3,20-dione

The title compound was prepared by hydrolysis of the compound prepared in Example 10a (245 mg, 0.5 mmol) in a mixture of methylenechloride (10 ml) and diethyl ether (40 ml). The reaction mixture was stirred with hydrochloric acid (20 ml) at 0°C for 1.5 hours. Yield: 125 mg containing 75% of the title compound, m.p. 190—195°C, after two crystallizations from methanol. (alpha)$^{20}$ +225° ($CHCl_3$, c 1.00). IR (Nujol*): 1665 (C=O), 1620, 1595 (C=C). $^1$H NMR ($CDCl_3$): delta 0.75—3.12 (m), 0.90 (s), 1.39 (s), 2.31 (s), 5.42—5.68 (m, 1H), 5.77 (s, 1H), 6.63—6.87 (m, 1H).

Example 11a

Preparation of 20-isocyano-20-p-methylphenylsulfonyl-21-benzyloxy-pregna-1,4,16-triene-3-one

The title compound was prepared according to the method described in Example 8a, starting from 17-(isocyano-p-methylphenylsulfonyl-methylene)-androsta-1,4-diene-3-one (462 mg, 1 mmol). Yield: 625 mg of a mixture of the title compound and chloromethyl benzylether (1:1). $^1$H NMR ($CDCl_3$): delta 0.57—3.00 (m), 0.80 (s), 1.01 (s), 1.19 (s), 2.38 (s), 3.69—4.26 (m, 2H), 4.53 (s, 2H), 5.92—6.51 (m, 3H), 6.80—7.51 (m, 8H), 7.70—7.84 (AB, 2H).

Example 11b

Preparation of 21-benzyloxy-pregna-1,4,16-triene-3,20-dione

The title compound was prepared according to the method described in Example 9b starting from the reaction mixture of Example 11a. Crystallization from methanol (−20°C) afforded 195 mg (47%) of the title

*Trade Mark

compound, m.p. 193—198°C (dec.). IR (Nujol*): 1660 (C=O), 1625, 1605, 1585 (C=C), 1110 (C—O—C). $^1$H NMR ($CDCl_3$): 0.62—2.93 (m), 1.00 (s), 1.27 (s), 4.35 (s, 2H), 4.59 (s, 2H), 6.05, 6.09, 6.27, 6.31 (2xd, 2H), 6.62—6.79 (m, 1H), 6.95, 7.12 (d, 1H), 7.28 (s, 5H).

### Example 12a

Preparation of 3-methoxy-20-isocyano-20-p-methylphenylsulfonyl-21-chloro-pregna-3,5,9(11),16-tetraene

3-Methoxy-17-(isocyano-p-methylphenylsulfonyl-methylene)-androsta-3,5,9(11)-triene (476 mg, 1.00 mmol) and triethylbenzylammonium chloride (20 mg) were dissolved in methylene chloride (10 ml). After stirring for 2 hours at 25°C with a sodium hydroxide solution (5 ml, 50%), the organic layer was separated and dried over $Na_2SO_4$, followed by filtration over alumina (act. II—III). Evaporation of the solvent afforded the title compound (368 mg, 70%). IR (Nujol*): 1150 (N=C), 1660, 1635, 1600 (C=C), 1340, 1155 ($SO_2$). $^1$H NMR ($CDCl_3$): delta 0.44—2.90 (m), 0.57 (s), 0.98 (s), 1.13 (s), 3.56 (s, 3H), 3.79—4.35 (s + m, 2H), 5.05—5.62 (m, 3H), 6.08—6.60 (m, 1H), 7.29, 7.44, 7.77, 7.92 (AB, 4H).

### Example 12b

Preparation of 21-chloro-pregna-4,9(11),16-triene-3,20-dione

The title compound was prepared according to the process described in Example 10b, starting with the reaction mixture of Example 12a. Yield: 145 mg of a mixture containing 65% of the title compound. IR (Nujol*): 1670 (C=O), 1620, 1595 (C=C). $^1$H NMR ($CDCl_3$): delta 0.62—3.09 (m), 0.90 (s), 1.36 (s), 4.38 (s, 2H), 5.22—5.88 (m, 2H), 6.74—6.92 (m, 1H).

### Example 13a

Preparation of 3-methoxy-20-isocyano-20-p-methylphenylsulfonyl-21-methyl-pregna-3,5,16-triene

The title compound was prepared according to the method described in Example 8a starting from 3-methoxy-17-(isocyano-p-methylphenylsulfonyl-methylene)-androsta-3,5-diene (954 mg, 2 mmol) and ethyl iodide (4.9 mmol). Yield: 900 mg (89%), m.p. 135—150°C. IR (Nujol*): 2170 (N=C), 1660, 1635, 1605 (C=C), 1340, 1160 ($SO_2$). $^1$H NMR ($CDCl_3$): delta 0.45—2.74 (m), 0.60 (s), 0.97 (s), 2.46 (s), 3.47 (s, 3H), 4.88—5.27 (m, 2H), 5.82—6.01, 6.20—6.39 (2xm, 1H), 7.08, 7.24, 7.58, 7.74 (AB, 4H).

### Example 13b

Preparation of 21-methyl-pregna-4,16-diene-3,20-dione

The title compound was prepared by hydrolysis of the compound prepared in Example 13a (253 mg, 0.5 mmol) according to the process described in Example 10b. Yield: 140 mg of a mixture containing 75% of the product. IR (Nujol*): 1665 (C=O), 1615, 1590 (C=C). $^1$H NMR ($CDCl_3$): delta 0.50—3.05 (m), 1.00 (s), 1.12 (t), 5.66 (s, 1H), 6.50—6.74 (m, 1H).

### Example 14a

Preparation of 3-methoxy-20-isocyano-20-p-methylphenylsulfonyl-21-chloro-19-nor-pregna-1,3,5(10),16-tetraene

The title compound was prepared according to the process described in Example 12a, starting with 3-methoxy-17-(isocyano-p-methylphenylsulfonyl-methylene)-estra-1,3,5(10)-triene (461 mg, 1 mmol). Yield: 5.10 mg. IR (Nujol*): 2160 (N=C). $^1$H NMR ($CDCl_3$): delta 0.6—3.2 (m), 0.65 (s), 1.01 (s), 1.51 (s), 2.48 (s), 3.77 (s, 3H), 3.9—4.2 (m, 2H), 6.05—6.25 (m, 0.5H), 6.45—6.85 (m, 0.5H), 7.0—7.6 (m), 7.81, 7.94 (½AB, 2H).

### Example 14b

Preparation of 3-methoxy-21-chloro-19-nor-pregna-1,3,5(10),16-tetraen-20-one

The title compound was prepared by hydrolysis of the reaction mixture of Example 14a (510 mg) in a mixture of methylene chloride (7.5 ml) and diethylether (20 ml). The reaction mixture was stirred with hydrochloric acid (10 ml, 36%) at 20°C for 10 minutes. Chromatography (diethylether, alumina act. II—III) afforded 100 mg of the title compound (29%), m.p. 134—141°C (from diethylether). IR (Nujol*): 1680 (C=O). $^1$H NMR ($CDCl_3$): delta 0.75—3.25 (m), 0.92 (s), 3.72 (s, 3H), 4.33 (s, 2H), 6.5—6.9 (m, 3H), 7.0—7.3 (m, 1H). Exact mass: 344.154 (calcd. 344.153).

### Example 15a

Preparation of 3-methoxy-20-isocyano-20-p-methylphenylsulfonyl-21-chloro-pregna-3,5,16-triene

The title compound was prepared according to Example 12a starting with 3-methoxy-17-(isocyano-p-methylphenylsulfonyl-methylene)-androsta-3,5-diene (477 mg, 1 mmol). Yield: 470 mg. IR (Nujol*): 2160 (N=C), 1655, 1630, 1595 (C=C), 1340—1155 ($SO_2$). $^1$H NMR ($CDCl_3$): delta 0.5—2.7 (m), 0.65 (s), 0.95 (s), 1.02 (s), 2.45 (s), 3.52 (s, 3H), 3.75—4.35 (m, 2H), 5.0—5.35 (m, 2H), 6.0—6.25 (m, 0.4H), 6.3—6.6 (m, 0.6H), 7.29, 7.42, 7.74, 7.88 (AB, 4H).

---

* Trade Mark

Example 15b

Preparation of 21-chloro-pregna-4,16-diene-3,20-dione

The title compound was prepared by hydrolysis of the compound prepared in Example 15a in a mixture of methylene chloride (5 ml) and diethyl ether (25 ml). This mixture was stirred vigorously with hydrochloric acid (2 ml, 38%), for 5 minutes. After work up and chromatography (methylene chloride, alumina act. II—III) the title compound was obtained (55 mg, 15%), IR (Nujol*): 1675 (C=O). $^1H$ NMR ($CDCl_3$): delta 0.7—2.7 (m), 0.89 (s), 1.21 (s), 4.34 (s, 2H), 5.73 (s, 1H), 6.65—6.90 (m, 1H), Exact mass: 346.169 (calcd. 346.170).

Example 16

Preparation of 3-methoxy-20-isocyano-20-p-methylphenylsulfonyl-21-bromo-19-nor-pregna-1,3,5(10),16-tetraene

The title compound was prepared according to the process described in Example 12a, starting with 3-methoxy-17-(isocyano-p-methylphenylsulfonyl-methylene)-estra-1,3,5(10)-triene (461 mg, 1 mmol) in a solution of dibromomethane (5 ml). Yield: 475 mg. IR (Nujol*): 2160 (N=C), 1340, 1160 ($SO_2$). $^1H$ NMR ($CDCl_3$): delta 0.6—3.2 (m), 0.69 (s), 0.97 (s), 1.07 (s), 2.46 (s), 3.7—4.2 (m, 5H), 3.73 (s), 3.90 (s), 6.05—6.25 (m, 0.2H), 6.4—6.9 (m, 2.2H), 7.08 (s, 1H), 7.28, 7.42, 7.79, 7.93 (AB, 4H).

Example 17a

Preparation of 3-methoxy-20-isocyano-20-p-methylphenylsulfonyl-21-bromo-pregna-3,5,16-triene

The title compound was prepared according to the process described in Example 16, starting with 3-methoxy-17-(isocyano-p-methylphenylsulfonyl-methylene)-androsta-3,5-diene (477 mg, 1 mmol). Methanol (20 ml) was added to the crude product and vibrated in a ultrasonic bath. The solid was sucked off and dried (product A, 247 mg). The mother liquid was evaporated yielding a second product (B, 190 mg). According to NMR the products appeared to be two stereoisomers of the title compound. IR (Nujol*): 2150 (N=C), 1655, 1630, 1600 (C=C), 1335, 1150 ($SO_2$). $^1H$ NMR ($CDCl_3$): product A, delta 0.5—2.7 (m), 0.65 (s), 0.95 (s), 2.44 (s), 3.51 (s, 3H), 3.86 (s, 2H), 5.0—5.35 (m, 2H), 6.3—6.6 (m, 1H), 7.27, 7.41, 7.73, 7.88 (ABq, 4H). $^1H$ NMR ($CDCl_3$): product B, delta 0.5—2.7 (m), 0.97 (s), 1.04 (s), 2.43 (s), 3.4—4.5 (m, 5H), 3.52 (s), 3.68 (s), 3.80 (s), 3.98 (s), 4.13 (s), 4.40 (s), 5.0—5.35 (m, 2H), 5.9—6.2 (m, 1H), 7.29, 7.43, 7.73, 7.88 (ABq, 4H).

Example 17b

Preparation of 21-bromo-pregna-4,16-diene-3,20-dione

The title product was obtained by hydrolysis of the product prepared in Example 17a. The hydrolysis was carried out with hydrobromic acid (6 ml, 48%) at 0°C during 1.5 hours. The reaction mixture was neutralized with $NaHCO_3$ and extracted with methylene chloride. After drying and evaporation in vacuo an oil was obtained. Chromatography over alumina with methylene chloride afforded 90 mg of the title compound (23%), m.p. 141—143°C (from diethylether). IR (Nujol*): 1660 (C=O). $^1H$ NMR ($CDCl_3$): delta 0.7—2.7 (m), 0.98 (s), 1.21 (s), 4.10 (s, 2H), 5.73 (s, 1H), 6.65—6.90 (m, 1H).

Example 18a

Preparation of 3-methoxy-20-isocyano-20-p-methylphenylsulfonyl-21-methoxy-pregna-3,5,16-triene

The title compound was prepared according to the process described in Example 8a, starting with 3-methoxy-17-(isocyano-p-methylphenylsulfonyl-methylene)-androsta-3,5-diene (477 mg, 1 mmol) and chloromethyl methyl ether (0.2 ml). Yield: 385 mg. IR (Nujol*): 2160 (N=C). $^1H$ NMR ($CDCl_3$): delta 0.5—3.0 (m), 0.79 (s), 1.00 (s), 1.04 (s), 2.45 (s), 3.39 (s, 3H), 3.56 (s, 3H), 3.95 (s, 2H), 5.05—5.40 (m, 2H), 6.05—6.30 (m, 0.3H), 6.35—6.60 (m, 0.7H), 7.30, 7.45, 7.80, 7.95 (AB, 4H).

Example 18b

Preparation of 21-methoxy-pregna-4,16-diene-3,20-dione

The organic phase of the reaction mixture of Example 18a was hydrolyzed with hydrochloric acid (7.5 ml, 25%). Chromatography over alumina (act. II—III) with methylene chloride afforded the title compound (130 mg, 38%). IR (Nujol*): 1685 (sh), 1665 (C=O), 1615 (C=C). $^1H$ NMR ($CDCl_3$) delta 0.6—2.8 (m), 0.97 (s), 1.21 (s), 3.40 (s, 3H), 4.28 (s, 2H), 5.72 (s, 1H), 6.65—6.85 (m, 1H). Exact mass 342.218 (calcd. 342.219).

Example 19a

Preparation of 3-methoxy-20-isocyano-20-p-methylphenylsulfonyl-21-methoxy-19-nor-pregna-1,3,5(10),16-tetraene

The title compound was prepared according to the process described in Example 8a, starting with 3-methoxy-17-(isocyano-p-methylphenylsulfonyl-methylene)-estra-1,3,5(10)-triene (461 mg, 1 mmol) and chloromethyl methyl ether. Yield: 355 mg brown oil. $^1H$ NMR ($CDCl_3$): delta 0.6—3.1 (m), 0.73 (s), 0.97 (s), 2.42 (s), 3.35, 3.38 (2xs, 3H), 3.70 (s, 3H), 3.97 (s, 2H), 6.0—6.9 (m, 3H), 7.0—7.5 (m, 2H), 7.80, 7.93 ($\frac{1}{2}$AB, 2H).

* Trade Mark

Example 19b

Preparation of 3,21-dimethoxy-19-nor-pregna-1,3,5(10),16-tetraene-20-one

The reaction product of Example 19a was dissolved in diethyl ether (40 ml) and methylene chloride (5 ml) and vigorously stirred with hydrochloric acid (5 ml, 38%). After work up and chromatography over alumina (act. II—III) with dichloromethane the title compound was obtained (50 mg, 15%), m.p. 95—100°C (from diethyl ether). IR (Nujol*): 1675 (C=O). $^1H$ NMR ($CDCl_3$): delta 0.8—3.2 (m), 0.95 (s), 3.42 (s, 3H), 3.75 (s, 3H), 4.30 (s, 2H), 6.5—6.9 (m, 3H), 7.13 (s, 1H). Exact mass 340.204 (calcd. 340.204).

**Claims**

1. Process for the preparation of 20-keto-delta$^{16}$-steroids of formula III:

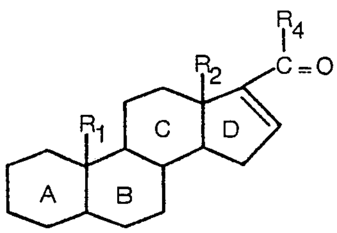

III

in which

$R_1$ represents a hydrogen atom or an alkyl group having 1 to 4 carbon atoms, or is absent in the case of a double bond between $C_{10}$ and $C_1$, $C_5$ or $C_9$,

$R_2$ represents a hydrogen atom or an alkyl group having 1 to 4 carbon atoms,

$R_4$ represents an alkyl group, optionally substituted by one or more halogen atoms, an alkoxy or phenylalkoxy group, a cycloalkyl group having 3—8 carbon atoms or a phenyl group optionaly substituted by one or more halogen atoms, alkyl, alkoxy, nitro or cyano groups and the rings A, B, C and D may contain one or more double bonds and may be substituted by one or more of the following groups: hydroxy, amino, oxo, halogen, alkyl, alkylene, alkoxy, alkoxyalkoxy, epoxy, methylene, alkylenedioxy, alkylenedithio or alkyleneoxythio, characterized by reacting a 17-(isocyano-sulfonyl-methylene)-steroid of formula I:

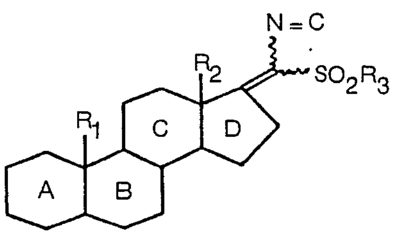

I

in which

$R_1$ and $R_2$ have the meanings as listed above and rings A, B, C and D may contain double bonds and may carry substituents as defined above and in which

$R_3$ represents an alkyl group, an optionally substituted aryl group or a dialkylamino group, wherein the alkyl groups together with the nitrogen atom may form a heterocyclic ring with up to 8 ring atoms and optionally containing an oxygen atom, under basic conditions with an alkylating agent $QR_4$, in which $R_4$ has the meaning as defined above and Q may be any group which is readily displaced by a nucleophile, followed by hydrolysis in an acidic aqueous solution.

2. Process for the preparation of 20-isocyano-20-sulfonyl-delta$^{16}$-steroids of formula II

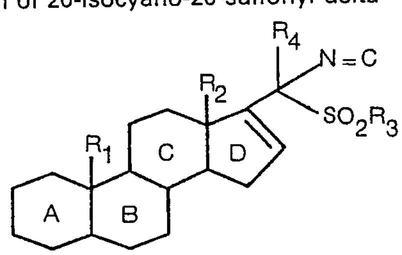

II

in which $R_1$, $R_2$, $R_3$ and $R_4$ have the meanings as defined in claim 1, rings A, B, C and D may contain double bonds and may carry substituents as defined in claim 1 characterized by reacting a 17-(isocyano-sulfonyl-methylene)-steroid according to claim 1 under basic conditions with an alkylating agent $QR_4$ as defined in claim 1.

* Trade Mark

3. Process for the preparation of 20-keto-delta$^{16}$-steroids according to claim 1, characterized by hydrolysis of the 20-isocyano-20-sulfonyl-delta$^{16}$-steroids according to claim 2 in the acidic aqueous solution.

4. Process according to claim 1 or 2, characterized in that the reaction is carried out in an inert organic solvent using a metal hydroxide, a metal alcoholate or a quaternary ammonium or phosphonium hydroxide as base.

5. Process according to claim 4, characterized in that a catalyst is added to the reaction mixture in the form of a quaternary ammonium or phosphonium salt or a crown ether.

6. Process according to claim 1 or 2, characterized in that the reaction is carried out under phasetransfer conditions.

7. Process according to any one of claims 1, 2 and 4—6, characterized in that Q is bromine, chlorine, iodine atom or a quaternary ammonium or sulfonate group.

8. Process according to any one of claims 1, 2 and 4—7, characterized in that $R_4$ is an alkyl group having 1—6 carbon atoms, substituted or not substituted by one or more halogen atoms, or an alkoxy or phenylalkoxy group, a cycloalkyl group having 3—8 carbon atoms or a phenyl group, substituted or not substituted by one or more halogen atoms or alkyl, alkoxy, nitro or cyano groups.

9. Process according to claim 8, characterized in that $R_4$ is methyl, halomethyl, particularly chloro-, bromo- or iodomethyl, methoxy or benzyloxy.

10. Process according to claim 1 or 3, characterized in that the hydrolysis is carried out in an organic solvent using an acidic aqueous solution.

11. Process according to any one of claims 1—3, characterized in that the 17-(isocyano-sulfonyl-methylene)-steroid has the general formula:

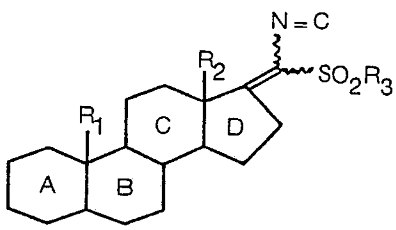

I

or the 20-isocyano-20-sulfonyl-delta$^{16}$-steroid has the general formula:

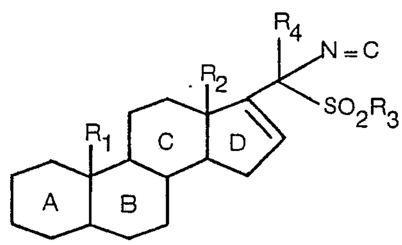

II

in which $R_1$ represents a hydrogen atom or a methyl group or is absent in the case of a double bond between $C_{10}$ and $C_1$, $C_5$ or $C_9$, $R_2$ represents a hydrogen atom or a methyl group, $R_3$ represents an alkyl or dialkylamino group, or an aryl group substituted or not substituted by one or more halogen atoms or alkyl, alkoxy, nitro or cyano groups, $R_4$ represents an alkyl group optionally containing one or more substituents as defined in claim 1, and the rings A, B, C and D optionally contain one or more double bonds, and are optionally substituted by one or more hydroxy groups, amino groups, oxygen atoms, halogen atoms or alkyl, alkylene, alkoxy or alkoxyalkoxy groups and are disubstituted or not disubstituted by one or more epoxy groups, methylene groups, alkylenedioxy, or alkylenedithio or alkyleneoxythio groups.

12. Process as defined in claim 11, characterized in that $R_3$ represents an alkyl group having 1—10 carbon atoms or $R_3$ represents a phenyl or naphthyl group optionally substituted by a halogen atom, one or more alkyl groups or an alkoxy group, preferably $R_3$ represents a phenyl, p-methoxyphenyl or p-methylphenyl group.

13. Process according to claim 11 or 12, in which the rings A, B, C and D contain one or more double bonds characterized in that these double bonds are present between $C_1$ and $C_2$, $C_3$ and $C_4$, $C_4$ and $C_5$, $C_5$ and $C_6$, $C_6$ and $C_7$, $C_9$ and $C_{11}$ and/or $C_{11}C_{12}$.

14. Process according to any one of claims 11—13, characterized in that the rings A, B, C and D are substituted by one or more 3-, 9-, 11-, 12- or 14-hydroxy group, and/or by an oxygen atom at $C_3$, $C_{11}$ or $C_{12}$ and/or by one or more 6-, 9- or 11-fluorine, chlorine or bromine atoms, and/or by a 1- or 6-methyl group, and/or by a 3-, 9- or 11-alkoxy group containing 1—4 carbon atoms, and/or by a 3- or 11-alkoxyalkoxy group.

15. Process according to any one of claims 11—14, in which the rings A, B, C and D are disubstituted by an epoxy group at $C_1$ and $C_2$ or $C_9$ and $C_{11}$ and/or by a methylene group attached to $C_1$ and $C_2$ and/or by a 3,3-alkylenedioxy, a 3,3-alkylenedithio or a 3,3-alkyleneoxythio group.

16. 20-Isocyano-20-sulfonyl-delta[16]-steroids with the general formula:

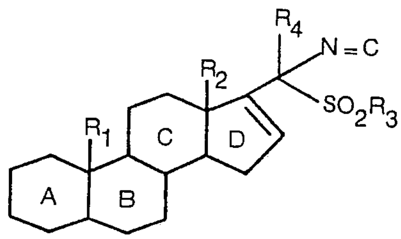

II

as defined in claims 11—15.

**Patentansprüche**

1. Verfahren zur Herstellung von 20-Keto-delta-16-steroiden der Formel III:

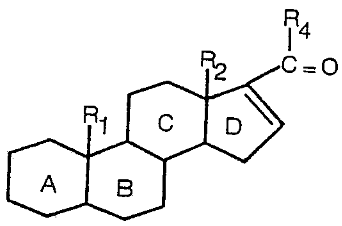

III

worin

$R_1$ ein Wasserstoffatom oder eine Alkylgruppe mit 1 bis 4 Kohlenstoffatomen bedeutet oder im Falle einer Doppelbindung zwischen $C_{10}$ und $C_1$, $C_5$ oder $C_9$ abwesend ist,

$R_2$ ein Wasserstoffatom oder eine Alkylgruppe mit 1 bis 4 Kohlenstoffatomen bedeutet,

$R_4$ eine Alkylgruppe, gegebenenfalls substituiert durch ein oder mehrere Halogenatome, eine Alkoxy- oder Phenylalkoxygruppe, eine Cycloalkylgruppe mit 3 bis 8 Kohlenstoffatomen oder eine Phenylgruppe, gegebenenfalls substituiert durch ein(e) oder mehrere Halogenatome, Alkyl-, alkoxy, Nitro- oder Cyanogruppen, bedeutet und die Ringe A, B, C und D eine oder mehrere Doppelbindungen enthalten können und durch eine oder mehrere der folgenden Gruppen substituiert sein können: Hydroxyl, Amino, Oxo, Halogen, Alkyl, Alkylen, Alkoxy, Alkoxyalkoxy, Epoxy, Methylen, Alkylendiox, Alkylendithio oder Alkylenoxythio, dadurch gekennzeichnet, dass man ein 17-(Isocyano-sulfonyl-methylen)-steroid der Formel I:

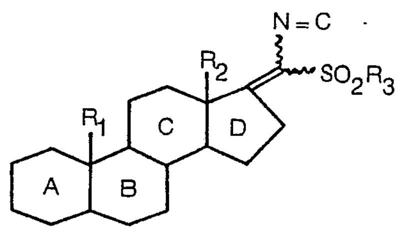

I

worin

$R_1$ und $R_2$ die oben aufgeführten Bedeutungen haben und die Ringe A, B, C und D Doppelbindungen enthalten können und wie oben definierte Substituenten tragen können und

$R_3$ eine Alkylgruppe, eine gegebenenfalls substituierte Arylgruppe oder eine Dialkylaminogruppe, worin die Alkylgruppen zusammen mit dem Stickstoffatom einen heterocyclischen Ring mit bis zu 8 Ringatomen, der gegebenenfalls ein Sauerstoffatom enthält, bilden können, beduetet, unter basischen Bedingungen mit einem Alkylierungsmittel $QR_4$, worin $R_4$ die oben definierte Bedeutung hat und Q eine beliebige Gruppe sein kann, die leicht durch ein Nucleophil erssetzt wird, umsetzt, gefolgt von Hydrolyse in einer saueren wässrigen Lösung.

2. Verfahren zur Herstellung von 20-Isocyano-20-sulfonyl-delta-16-steroiden der Formel II:

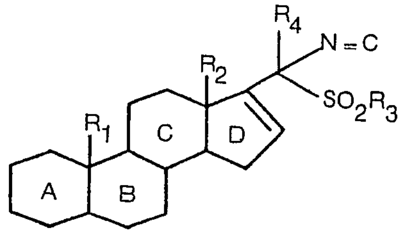

II

worin $R_1$, $R_2$, $R_3$ und $R_4$ die in Anspruch 1 definierten Bedeutungen haben und die Ringe A, B, C und D Doppelbindungen enthalten können und wie in Anspruch 1 definierte Substituenten tragen können, gekennzeichnet durch Umsetzung eines 17-(Isocyano-sulfonyl-methylen)-steroides gemäss Anspruch 1 unter basischen Bedingungen mit einem wie in Anspruch 1 definierten Alkylierungsmittel $QR_4$.

3. Verfahren zur Herstellung von 20-Keto-delta-16-steroiden gemäss Anspruch 1, gekennzeichnet durch Hydrolyse der 20-Isocyano-20-sulfonyl-delta-16-steroide gemäss Anspruch 2 in einer sauren wässrigen Lösung.

4. Verfahren nach Anspruch 1 oder 2, dadurch gekennzeichnet, dass man die Reaktion in einem inerten organischen Lösungsmittel unter Verwendung eines Metallhydroxides, eines Metallalkoholates oder eines quaternären Ammonium- oder Phosphoniumhydroxides als Base ausführt.

5. Verfahren nach Anspruch 4, dadurch gekennzeichnet, dass man einer Katalysator in Form eines quaternären Ammonium- oder Phosphoniumsalzes oder eines Kronenethers zu dem Reaktions-gemisch zusetzt.

6. Verfahren nach Anspruch 1 oder 2, dadurch gekennzeichnet, dass man die Reaktion unter Phasentransferbedingungen ausführt.

7. Verfahren nach einem der Ansprüche 1, 2 und 4 bis 6, dadurch gekennzeichnet, dass Q ein Brom-, Chlor- oder Iodatom oder eine quaternäre Ammonium- oder Sulfonatgruppe ist.

8. Verfahren nach einem der Ansprüche 1, 2 und 4 bis 7, dadurch gekennzeichnet, dass $R_4$ eine Alkylgruppe mit 1 bis 6 Kohlenstoffatomen, substituiert oder nicht substituiert durch ein oder mehrere Halogenatome oder eine Alkoxy- oder Phenylalkoxygruppe, eine Cycloalkylgruppe mit 3 bis 8 Kohlenstoffatomen oder eine Phenylgruppe, substituiert oder nicht substituiert durch ein(e) oder mehrere Halogenatome oder Alkyl-, Alkoxy-, Nitro- oder Cyanogruppen, ist.

9. Verfahren nach Anspruch 8, dadurch gekennzeichnet, dass $R_4$ Methyl, Halogenmethyl, insbesondere Chlor-, Brom- oder Iodmethyl, Methoxy oder Benzyloxy ist.

10. Verfahren nach Anspruch 1 oder 3, dadurch gekennzeichnet, dass man die Hydrolyse in einem organischen Lösungsmittel unter Verwendug einer sauren wässrigen Lösung ausführt.

11. Verfahren nach einem der Ansprüche 1 bis 3, dadurch gekennzeichnet, dass das 17-(Isocyanosulfonyl-methylen)-steroid die allgemeine Formel:

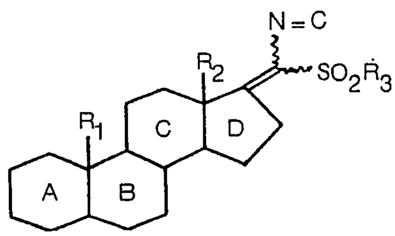

I

hat oder das 20-Isocyano-20-sulfonyl-delta-16-steroid die allgemeine Formel:

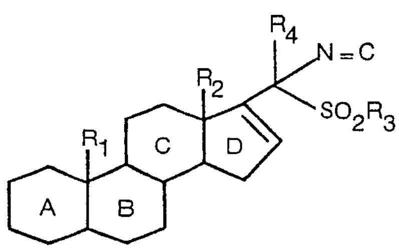

II

hat, worin $R_1$ ein Wasserstoffatom oder eine Methylgruppe bedeutet oder im Falle einer Doppelbindung zwischen $C_{10}$ und $C_1$, $C_5$ oder $C_9$ abwesend ist, $R_2$ ein Wasserstoffatom oder eine Methylgruppe bedeutet, $R_3$ eine Alkyl- oder Dialkylaminogruppe oder eine Arylgruppe, substituiert oder nicht substituiert durch ein(e) oder mehrere Halogenatom oder Alkyl-, alkoxy-, Nitro- oder Cyanogruppen, bedeutet, $R_4$ eine Alkylgruppe, die gegebenenfalls einen oder mehrere wie in Anspruch 1 definierte Substituenten enthält, bedeutet und die Ringe A, B, C und D gegebenenfalls eine oder mehrere Doppelbindungen enthalten und gegebenenfalls durch ein(e) oder mehrere Hydroxylgruppen, Aminogruppen, Sauerstoffatome, Halogenatome oder Alkyl-, Alkylen-, Alkoxy- oder Alkoxyalkoxygruppen substituiert sind und durch eine oder mehrere Epoxygruppen, Methylengruppen, Alkylendioxy- oder Alkylendithio- oder alkylenoxythiogruppen disubstituiert oder nicht disubstituiert sind.

12. Verfahren nach Anspruch 11, dadurch gekennzeichnet, dass $R_3$ eine Alkylgruppe mit 1 bis 10 Kohlenstoffatomen bedeutet oder $R_3$ eine Phenyl- oder Naphthylgruppe, die gegebenenfalls durch ein Halogenatom, eine oder mehrere Alkylgruppen oder ein Alkoxygruppe substituiert ist, bedeutet, wobei $R_3$ vorzugsweise eine Phenyl-, p-Methoxyphenyl- oder p-Methylphenylgruppe bedeutet.

13. Verfahren nach Anspruch 11 oder 12, worin die Ringe A, B, C und D eine oder mehrere Doppelbindungen enthalten, dadurch gekennzeichnet, dass diese Doppelbindungen zwischen $C_1$ und $C_2$, $C_3$ und $C_4$, $C_4$ und $C_5$, $C_5$ und $C_6$, $C_6$ und $C_7$, $C_9$ und $C_{11}$ und/oder $C_{11}$ und $C_{12}$ vorliegen.

14. Verfahren nach einem der Ansprüche 11 bis 13, dadurch gekennzeichnet, dass die Ringe A, B, C und D durch eine oder mehrere 3-, 9-, 11-, 12- oder 14-Hydroxylgruppen und/oder durch ein Sauerstoffatom am $C^3$, $C_{11}$ oder $C_{12}$ und/oder durch ein oder mehrere 6-, 9- oder 11-Fluor-, -Chlor- oder -Brom-atome und/oder

durch eine 1- oder 6-Methylgruppe und/oder durch eine 3-, 9- oder 11-Alkoxygruppe, die 1 bis 4 Kohlenstoffatome enthält, und/oder durch eine 3- oder 11-Alkoxyalkoxygruppe substituiert sind.

15. Verfahren nach einem der Ansprüche 11 bis 14, worin die Ringe A, B, C und D durch eine Epoxygruppe an $C_1$ und $C_2$ oder $C_9$ und $C_{11}$ und/oder durch eine Methylengruppe, die an $C_1$ und $C_2$ gebunden ist, und/oder durch eine 3,3-Alkylendioxy-, eine 3,3-Alkylendithio- oder eine 3,3-Alkylenoxythiogruppe disubstituiert sind.

16. 20-Isocyano-20-sulfonyl-delta-16-steroide mit der allgemeinen Formel:

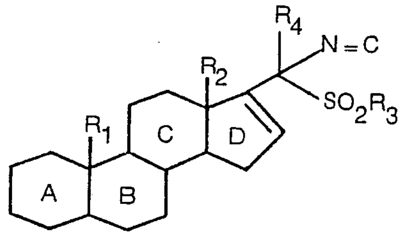

II

wie in den Ansprüchen 11 bis 15 definiert.

**Revendications**

1. Procédé de préparation de 20-céto-delta$^{16}$-stéroïdes de formule III:

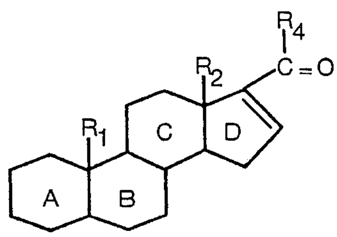

III

où $R_1$ représente un atome d'hydrogène ou un radical alcoyle de 1 à 4 atomes de carbone ou bien est absent dans le cas d'une double liaison entre $C_{10}$ et $C_1$, $C_5$ ou $C_9$,

$R_2$ représente un atome d'hydrogène ou un radical alcoyle de 1 à 4 atomes de carbone,

$R_4$ représente un radical alcoyle éventuellement substitué par un ou plusieurs atomes d'halogène, un radical alcoxy ou phénylalcoxy, un radical cycloalcoyle de 3 à 8 atomes de carbone ou un radical phényle éventuellement substitué par un ou plusieurs atoms d'halogène ou radicaux alcoyle, alcoxy, nitro ou cyano, et les cycles A, B, C et D peuvent contenir une ou plusieurs doubles liaisons et peuvent être substitués par un ou plusieurs des radicaux suivants: hydroxyle, amino, oxo, halogène, alcoyle, alcoylène, alcoxy, alcoxyalcoxy, époxy, méthylène, alcoylènedioxy, alcoylènedithio ou alcoylèneoxythio, caractérisé par la réaction d'un 17-(isocyano-sulfonylméthylène)-stéroïde de formule I:

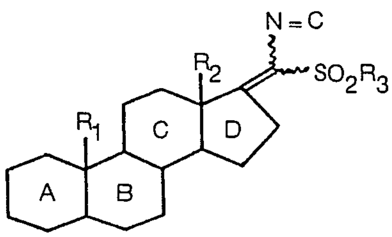

I

où

$R_1$ et $R_2$ ont les significations indiquées ci-dessus et les cycles A, B, C et D peuvent contenir des doubles liaisons et porter des substituants tels que définis ci-dessus, et

$R_3$ représente un radical alcoyle, un radical aryle éventuellement substitué ou un radical dialcoylamino, dont les radicaux alcoyle conjointement avec l'atome d'azote peuvent former un radical hétérocyclique comptant jusqu'à 8 atoms de cycle et contenant éventuellement un atome d'oxygène, dans des conditions basiques, avec un agent d'alcoylation

$$QR_4$$

où

$R_4$ a la signification indiquée ci-dessus, et

Q peut être un radical quelconque qui est aisément déplacé par un réactif nucléophile, suivie de l'hydrolyse dans une solution aqueuse acide.

2. Procédé de préparation de 20-isocyano-20-sulfonyl-delta$^{16}$-stéroïdes de formule II

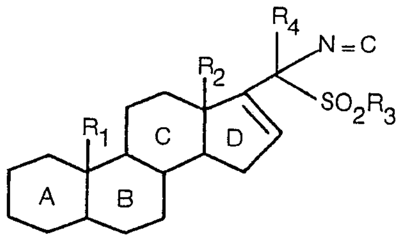

II

où $R_1$, $R_2$, $R_3$ et $R_4$ ont les significations telles que définies dans la revendication 1, et les cycles A, B, C et D peuvent contenir des doubles liaisons et porter des substituants tels que définis dans la revendication 1, caractérisé par la réaction d'un 17-(isocyano-sulfonylméthylène)-stéroïde suivant la revendication 1, dans des conditions basiques, avec un agent d'alcoylation $QR_4$ tel que défini dans la revendication 1.

3. Procédé de préparation de 20-céto-delta$^{16}$-stéroïdes suivant la revendication 1, caractérisé par l'hydrolyse des 20-isocyano-20-sulfonyl-delta$^{16}$-stéroïdes suivant la revendication 2 dans une solution aqueuse acide.

4. Procédé suivant la revendication 1 ou 2, caractérisé en ce que la réaction est exécutée dans un solvant organique inerte au moyen d'un hydroxyde de métal, d'un alcoolate de métal ou d'un hydroxyde d'ammonium ou de phosphonium quaternaire comme base.

5. Procédé suivant la revendication 4, caractérisé en ce qu'un catalyseur est ajouté au mélange de réaction sous la forme d'un sel d'ammonium ou de phosphonium quaternaire ou d'un éther couronne.

6. Procédé suivant la revendication 1 ou 2, caractérisé en ce que la réaction est exécutée dans des conditions de transfert de phase.

7. Procédé suivant l'une quelconque des revendications 1, 2 et 4 à 6, caractérisé en ce que Q représente un atome de brome, de chlore ou d'iode ou un radical ammonium quaternaire ou sulfonate.

8. Procédé suivant l'une quelconque des revendications 1, 2 et 4 à 7, caractérisé en ce que $R_4$ représente un radical alcoyle de 1 à 6 atomes de carbone, non substitué ou substitué par un ou plusieurs atomes d'halogène, ou un radical alcoxy ou phénylalcoxy, un radical cycloalcoyle de 3 à 8 atomes de carbone ou un radical phényle non substitué ou substitué par un ou plusieurs atomes d'halogène ou radicaux alcoyle, alcoxy, nitro ou cyano.

9. Procédé suivant la revendication 8, caractérisé en ce que $R_4$ représente un radical méthyle, halométhyle, en particulier chloro-, bromo- ou iodométhyle, méthoxy ou benzyloxy.

10. Procédé suivant la revendication 1 ou 3, caractérisé en ce que l'hydrolyse est exécutée dans un solvant organique au moyen d'une solution aqueuse acide.

11. Procédé suivant l'une quelconque des revendications 1 à 3, caractérisé en ce que le 17-(isocyano-sulfonylméthylène)-stéroïde est de formule générale:

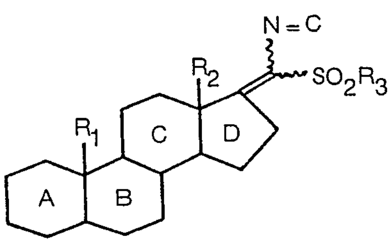

I

ou le 20-isocyano-20-sulfonyl-delta$^{16}$-stéroïde est de formule générale:

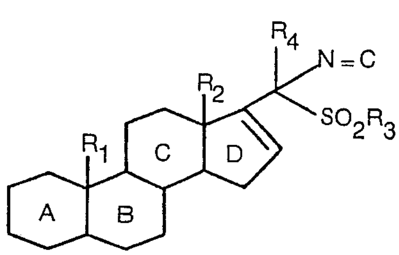

II

où

$R_1$ représente un atome d'hydrogène ou un radical méthyle ou bien est absent dans le cas d'une double liaison entre $C_{10}$ et $C_1$, $C_5$ ou $C_9$,

$R_2$ représente un atome d'hydrogène ou un radical méthyle,

$R_3$ représente un radical alcoyle ou dialcoylamino ou un radical aryle non substitué ou substitué par un ou plusieurs atomes d'halogène ou radicaux alcoyle, alcoxy, nitro ou cyano,

$R_4$ représente un radical alcoyle contenant éventuellement un ou plusieurs substituants tels que définis dans la revendication 1, et les cycles A, B, C et D contiennent éventuellement une ou plusieurs doubles liaisons et sont éventuellement substitués par un ou plusieurs radicaux hydroxyle, radicaux amino, atomes d'oxygène, atomes d'halogène ou radicaux alcoyle, alcoylène, alcoxy ou alcoxyalcoxy et sont disubstitués ou non disubstitués par un ou plusieurs radicaux époxy, radicaux méthylène ou radicaux alcoylènedioxy, alcoylènedithio ou alcoylèneoxythio.

12. Procédé suivant la revendication 11, caractérisé en ce que $R_3$ représente un radical alcoyle de 1 à 10 atomes de carbone ou $R_3$ représente un radical phényle ou naphtyle éventuellement substitué par un atome d'halogène, un ou plusieurs radicaux alcoyle ou un radical alcoxy et de préférence $R_3$ représente un radical phényle, p-méthoxyphényle ou p-méthylphényle.

13. Procédé suivant la revendication 11 ou 12, dans lequel les cycles A, B, C et D contiennent une ou plusieurs doubles liaisons, caractérisé en ce que ces doubles liaisons sont présentes entre $C_1$ et $C_2$, $C_3$ et $C_4$, $C_4$ et $C_5$, $C_5$ et $C_6$, $C_6$ et $C_7$, $C_9$ et $C_{11}$ et/ou $C_{11}$ et $C_{12}$.

14. Procédé suivant l'une quelconque des revendications 11 à 13, caractérisé en ce que les cycles A, B, C et D sont substitués par un ou plusieurs radicaux 3-, 9-, 11-, 12- ou 14-hydroxyle et/ou par un atome d'oxygène en $C_3$, $C_{11}$ ou $C_{12}$ et/ou par un ou plusieurs atomes de 6-, 9- ou 11-fluor, -chlore ou -brome, et/ou par un radical 1- ou 6-méthyle, et/ou par un radical 3-, 9- ou 11-alcoxy comptant 1 à 4 atomes de carbone et/ou par un radical 3- ou 11-alcoxyalcoxy.

15. Procédé suivant l'une quelconque des revendications 11 à 14, caractérisé en ce que les cycles A, B, C et D sont disubstitués par un radical époxy en $C_1$ et $C_2$ ou $C_9$ et $C_{11}$ et/ou par un radical méthylène uni en $C_1$ et $C_2$ et/ou par un radical 3,3-alcoylènedioxy un radical 3,3-alcoylènedithio ou un radical 3,3-alcoylèoxythio.

16. 20-Isocyano-20-sulfonyl-delta[16]-stéroïdes de la formule générale

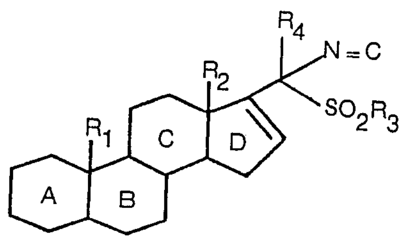

II

tels que définis dans les revendications 11 à 15.